# EUROPEAN PATENT APPLICATION

(11) **EP 1 767 220 A1**
(43) Date of publication of application: **28.03.2007**
(21) Application number: 06254983.7
(22) Date of filing: 26.09.2006
(51) Int. Cl.: A61K 47/46, A61K 9/00, A61K 36/00

(54) **Eriodictyon extract containing pharmaceuticals for nasal administration**

(30) Priority: 26.09.2005 US 720797 P; 26.07.2006 US 460194
(71) Applicant: Parnell, Francis W., Ross CA 94967 (US)
(72) Inventor: Parnell, Francis W., Ross CA 94967 (US)
(74) Representative: Couchman, Jonathan Hugh

(57) **Abstract**

Uses, compositions and systems are provided for the non-invasive transnasal and transocular drug delivery to the central nervous system using eriodictyon fluid extract technology. By administration through the olfactory nerve or the optical nerve, the delivery of a biologically active substance of interest into the CNS and CSF can be enhanced through bypassing the blood-brain barrier. The invention involves the use of eriodictyon fluid extract as an excipient in compositions and systems for administering drugs to the olfactory or optical nerve.

## Description

### BACKGROUND OF THE INVENTION

It is well known to administer drugs to or through the skin or mucosa. Such a mode of delivery provides many advantages; primarily, topical, transdermal or transmucosal delivery are generally comfortable, convenient and noninvasive ways of administering drugs. The variable rates of absorption and metabolism encountered with oral treatment are avoided, and other inconveniences--gastrointestinal irritation and the like--are eliminated as well.

A number of problems have been encountered with the aforementioned modes of drug administration, however. The skin or mucosa can become irritated or sensitized by a particular drug, adhesive or skin permeation enhancer. Pain and itching may result and be serious enough that the patient may discontinue use of the drug. In addition, the skin may become dry or flaky upon continued administration of a particular pharmaceutical composition. If such problems persist and are severe enough in a large fraction of patients, a particular drug may simply be designated as unsuitable for administration to the skin or mucosa, even though therapeutically effective blood levels may have been achieved by administering the drug in this way.

The present invention is premised on the completely unexpected discovery that the oil extracted from the Yerba Santa plant (Eriodictyon californicum; Eriodictyon glutinosum; also known as "eriodictyon fluid extract"; "consumptive's weed"; "bear's weed"; "mountain balm"; and "gum plant") is extremely effective as an excipient in compositions for application to the skin or mucosa, and minimizes or completely eliminates the irritation, sensitization and dryness which often accompanies topical, transdermal or transmucosal drug delivery. The novel excipient also makes possible the administration of a wider range of drugs than previously believed possible, i.e., drugs which caused a significant degree of the aforementioned problems in a relatively large fraction of patients.

The Yerba Santa plant is an evergreen shrub indigenous to the hills and mountains of California and northern Mexico, and was long used by Indians for a number of purposes. See, e.g., A. R. Hutchens, Indian Herbalogy of North America, Ontario: Merco, 1975, at pp. 317-318. A number of references to the Yerba Santa plant teach its use as an expectorant (e.g., N. Coon, The Dictionary of Useful Plants, Emmaus, Pa.: Rodale Press, 1974)), in treating colds, sore throats, catarrh, stomach aches, vomiting and diarrhea (see A. R. Hutchens, supra), in treating hemorrhoids (D. G. Spoerke, Herbal Medications, Santa Barbara, Calif.: Woodbridge Press, 1980, at p. 183), in treating diseases of the lung (Los Remedios de la Gente: A Compilation of Traditional New Mexican Herbal Medicines and Their Use, compiled by M. Moore, 1977), and in masking the taste of quinine and other bitter medications (Spoerke, supra; see also G. E. Trease et al., Pharmacognosy, London: Cassell & Colber, 1978, at p. 463)). References related to the Yerba Santa plant, in addition to the Coon, Hutchens, Moore, Spoerke, and Trease et al. references cited in the preceding section, include V. J. Vogel, American Indian Medicine, The University of Oklahoma Press, 1970, at pp. 83, 399-400; W. H. Lewis et al., Medical Botany: Plants Affecting Man's Health, New York: John Wiley & Sons, 1977, at p. 301; P. Huson, Mastering Herbalism: A Practical Guide, New York: Stein and Day, 1974, at p. 32; B. C. Harris, The Compleat Herbal, Barre, Mass.; Barre Publishers, 1972, at p. 197; N. Coon, Using Plants for Healing, Hearthside Press, 1963, at p. 122; M. Grieve, A Modem Herbal, vol. 22, New York: Hafner publishing Co., 1969, at p. 865; and V. E. Tyler et al., Pharmacognosy, Philadelphia: Lee & Febiger, 1981, at p. 148.

### BRIEF SUMMARY OF THE INVENTION

Most of the efforts currently under way to discover new therapeutic drugs for disorders of the central nervous system (CNS) face the problem of delivering the drugs to the brain without impairing the activity or integrity of such substances or compounds, while minimizing systemic adverse effects. And that means finding a way around--or through--the blood brain barrier (BBB), the physiological barrier between bloodstream and brain.

Accordingly, it is a primary object of the present invention to address the above-identified need in the art, and to provide uses, compositions and drug delivery systems for transmucosal drug administration and typically nasal administration, e.g. to the olfactory nerve, which involve the use of eriodictyon fluid extract as an excipient or vehicle.

Intranasal administration of the medication to the olfactory nerve provides an advantage. The olfactory nerve provides a direct connection between the outside environment and the brain thus providing quick and ready delivery of active agents for treatment of various disorders, however, delivery of drugs to the olfactory nerve must be approached with caution as the nasal mucosa in general and the olfactory neuroepithelium in particular are much more delicate and susceptible to damage than skin. Furthermore, the olfactory neuroepithelium is a very specialized type of epithelium that has a limited surface area and a poor regeneration potential. Thus, formulations for delivery to the olfactory nerve normally should be minimally irritating. Furthermore, it is generally desired that the formulations deliver the drug in a highly effective manner, thereby minimizing the number of applications necessary and reducing the opportunities for irritation of the olfactory nerve.

The uses and compositions of the invention are based on the unexpected finding that eriodictyon fluid extract is extremely effective in ameliorating the problems associated with delivery of drugs to the mucosa. Thus, formulations for delivery of active agents to the olfactory nerve, which are formulated with eriodictyon fluid extract have unexpectedly beneficial properties, including being less irritating to the mucosa and more effectively delivering the active agent to the desired site due to the ability of the extract to adhere to the surface of the olfactory nerve.

In one aspect, the invention encompasses pharmaceutical formulations containing a drug and eriodictyon-based excipient. These formulations may be for nasal administration. The formulations may be for delivery of a drug to a mammalian nervous system. The drug may be for treating a disorder of the nervous system, particularly the central nervous system. These formulations may at their simplest contain eriodictyon fluid as the sole source of active principle but are more usually a mixture of drug and eriodictyon fluid extract, wherein the eriodictyon fluid extract serves as an excipient, or they may be mixtures of drug, eriodictyon-based excipient, and one or more vehicles, carriers, diluents, permeation enhancers, or the like. The composition may be in the form of a solution, suspension, ointment, gel, cream, etc but is normally adapted for nasal administration.

In another aspect of the invention, a system is provided for delivering a drug to the olfactory nerve, the system comprising:
(a) a source of the drug to be administered;
(b) a source of an excipient composition comprising eriodictyon fluid extract; and
(c) a pressurized delivery device, configured to deliver the drug and excipient to the olfactory nerve.

In still another aspect, the invention is directed to the use of an eriodictyon fluid extract for the manufacture of a medicament for facilitating adherence of an agent to the olfactory nerve.The agent is applied to the olfactory nerve in conjunction with an excipient composition containing eriodictyon fluid extract. This aspect of the invention is based on the discovery that eriodictyon fluid extract is useful as a bioadhesive, i.e., a species which will increase adhesion of a device (e.g., a transdermal drug delivery device) or chemical species (such as a drug in an ointment, gel, solution or suspension) to the skin or mucosal tissue.

Aspects and embodiments of the present invention are set forth in the detailed description and claims that follow..

### DETAILED DESCRIPTION OF THE INVENTION

Before describing the present compositions, systems and uses of the invention in detail, it is to be understood that this invention is defined by the claims and that the scope of protection is not otherwise limited to the particular drugs, delivery systems, or dosage regimens described herein, as such may, of course, vary. It is also to be understood that the terminology used herein is for the purpose of describing particular embodiments only, and is not intended to be limiting since the scope of the present invention will be limited only by the appended claims.

It must be noted that, as used in this specification and the appended claims, the singular forms "a," "an" and "the" include plural referents unless the context clearly dictates otherwise. Thus, for example, reference to a laminated structure containing "a drug" includes a mixture of two or more drugs, reference to "an adhesive" includes reference to one or more of such adhesives, and reference to "a solvent" includes reference to a mixture of two or more solvents.

Other definitions of words and phrases used throughout this specification and the appended claims are as follows.

The term "drug" as used herein is intended to mean a compound or composition of matter which, when administered to an organism (human or animal) induces a desired pharmacologic and/or physiologic effect by local and/or systemic action. In general, the terms include the therapeutic or prophylactic agents in all major therapeutic/prophylactic areas of medicine. Examples of drugs useful in conjunction with the present invention include: anti-infectives such as antibiotics and antiviral agents; analgesics and analgesic combinations; anorexics; anticholinergic agents; anticonvulsants; antidepressants; antidiabetic agents; antihistamines; anti-inflammatory agents, antimigraine preparations; anti-motion sickness drugs; antinauseants; antineoplastics; antiparkinsonism drugs; antipruritics; antipsychotics; antipyretics; antispasmodics; sympathomimetics; xanthine derivatives; cardiovascular preparations including calcium channel blockers and beta-blockers such as pindolol and antiarrhythmics; antihypertensives; diuretics; vasodilators including general coronary, peripheral and cerebral; central nervous system stimulants; cough and cold preparations, including decongestants; steroids; hypnotics; immunosuppressives; muscle relaxants; parasympatholytics; psychostimulants; sedatives; tranquilizers; and agents for alleviating drug withdrawal symptoms.

The term "effective amount" as used herein intends that quantity of material which, when used or administered to a patient as indicated, is sufficient to provide the desired or intended beneficial effect. An "effective amount" of eriodictyon fluid extract to alleviate chemically induced irritation, e.g., irritation resulting from topical, transdermal or transmucosal administration of a drug, is an amount which is sufficient to provide a desired degree of relief. Again, the "effective amount" in this latter case will vary with the individual, the degree of irritation, and possibly with the particular type of chemical or drug, if any, which is inducing the irritation.

By "eriodictyon fluid extract" or "Yerba Santa fluid extract" as used herein is meant the fluid which may be extracted from dried Yerba Santa leaves. One exemplary method for obtaining this Yerba Santa fluid extract is set forth in Remington's Pharmaceutical Sciences, 17th Ed., 1985, at pp. 1286 and 1516, which is incorporated herein by reference to disclose such an extraction method. As described in detail in the aforementioned reference, the dried Yerba Santa plant is preferably processed in alcohol and water, followed by straining, pressing and clarification by, for example, decantation or filtration.

"Carriers" or "vehicles" as used herein refer to carrier materials suitable for transdermal, transmucosal or topical drug administration, and include any such materials known in the art, e.g., any liquid, gel, solvent, liquid diluent, solubilizer, or the like, which is nontoxic and which does not interact with other components of the particular composition or system in a deleterious manner. Examples of suitable carriers for use herein include water, mineral oil, silicone, nontoxic organic solvents, petroleum jelly, and a variety of other materials. The particular carrier or vehicle selected will depend on the particular system used, the desired rate of drug administration, as well as on the particular drug and its solubility in various solvents.

"Permeation-enhancement agent" refers to excipients in a composition for intranasal drug administration that enhance overall delivery, dose consistency and/or pharmaceutical acceptance. The methods for assessing enhancement are quantitative and are defined in the examples herein. Functional in vitro cell-based testing and methods are used to compare the effects of drug molecule by itself versus the same mass of drug molecule formulated and administered with excipients. "Enhancement" is a multivariant value proposition encompassing total bioavailability, net uptake, consistency of uptake and dosing, drug metabolism, degradation during dosing, drug targeting to active site(s), mucosal irritation, and overall safety and toxicity. In some cases, the relative benefit of the drug is used to optimize the excipient composition of the formulation, even at the expense of factors such as comfort and side effects. As explained when "pharmaceutical acceptance" was defined, the benefits of therapy may outweigh the discomfort of delivery. Chronic administration must also be considered. However, comfort and safety are optimized when overall delivery is not unacceptably compromised. As an illustration of the balance, pH may improve the stability of a drug and its transmucosal flux, but a formulation pH outside the range of pH 3 to 8.5 may result in pain and tissue damage when administered nasally.

### Introduction

It has long been known that the bulk of the brain and the spinal cord is surrounded by a specially secreted clear fluid called the cerebrospinal fluid (CSF). Chemical substances such as metabolites move relatively freely from the alimentary canal into the blood, but not into the CSF. As a result, the blood levels of sugars, amino acids or fatty acids fluctuate over wide range while their concentrations in the CSF remain relatively stable. The same is true for hormones, antibodies, certain electrolytes, and a variety of drugs. Injected directly into the blood, they act rapidly on peripheral tissues such as the muscles, heart, or glands but they have little or no effect on the central nervous system (CNS).

When administered into the CSF, however, the same substances exert a prompt and strong action. The conclusion is that the substances injected into the blood do not reach the CSF and the brain with sufficient rapidity and in an effective concentration.

The way in which the brain keeps its environment constant is frequently discussed in terms of a blood-brain barrier (BBB). Once substances have found their way into the CSF, they are free to diffuse into the tissues of the brain. The entry of hydrophilic and relatively large molecules into the CNS is restricted by the existence of a BBB. The BBB separates the brain from the blood circulation and is involved in the homeostasis of the brain. The BBB is situated in the brain microvessels and is composed of various cell types like endothelial cells, astrocytes, microglial cells, perivascular macrophages, and pericytes. The cerebral and endothelial cells form the morphological and functional basis of the BBB.

The olfactory area is located high in the nasal vault above the superior turbinate. Sensory hairs extend from the surface of the olfactory area to the cells that lie deep in the mucosa. Nerve fibers subserving the sense of smell have their cells of origin in the mucous membrane of the upper and posterior parts of the nasal cavity. The entire olfactory mucosa covers an area of about 2.5 cm². The central processes of olfactory fila are very fine unmyelinated fibers that converge to form small fascicles enwrapped by Schwann cells and pass through openings in the cribriform plate of the ethmoid bone into the olfactory bulb. The axons of the mitral and tufted enter the olfactory tract, which courses along the olfactory groove of the cribriform plate to the cerebrum. Some fibers project to the medial dorsal nucleus of the thalamus and the hypothalamus. That olfactory stimuli and emotional stimuli are strongly linked is not surprising, in view of their common roots in the limbic system.

According to Bell, the olfactory system has direct neuroanatomical and neurophysiological input to the amygdala and eventually hippocampus. Therefore it is conceivable that chemical stimuli at low levels could trigger limbic dysfunction in patients who happen to meet descriptive criteria for somatization disorder. It is also stated that there is no blood-brain barrier in the nasal passages. The limbic structure (e.g. the amygdala, olfactory bulb and hippocampus) can become easily kindled. The olfactory nerves can transport toxins directly to the limbic system. This may result in symptoms including memory loss, irritable bowel, and migraine headaches.

It has been suggested by Shipley that it is possible to transport substances which come into contact with the nasal epithelium to the brain and that it is thus possible to influence the function of neurons in the brain, including some which have extensive projection to wide areas of the CNS.

The optic nerve, mediating vision, is distributed to the eyeball. Most of its fibers are afferent and originate in the nerve cells of the ganglionic layer of the retina. Developmentally, the optic nerves and the retinae are parts of the brain and their fibers with glia.

The optic nerve, about 4 cm long, is directed backwards and medially through the posterior part of the orbital cavity. It then runs through the optic canal into cranial cavity and joins the optic chiasma. The optic nerve is enclosed in three sheaths, which are continuous with the membranes of the brain, and are prolonged as far as the back of the eyeball. Therefore, there is a direct connection between the optic nerve and the brain structures.

Itaya and van Hoesen described transneuronal retrograde labeling of neurons in the stratum griseum superficiale of the superior colliculus following intraocular injection of wheat germ agglutinin-horseradish peroxidase. A study of the distribution of wheat germ agglutinin-horseradish peroxidase in the visual system following intraocular injections in the chick, rat and monkey confirmed early findings of transneural transport of this conjugate in vivo.

From the overview of the literature given above we can conclude the following. Many substances such as metabolic products, drugs, and other substances cannot or only to a limited extent cross the BBB from the blood into the brain. From the nasal cavity these substances can penetrate into the brain because in the area of 2.5 cm² of the upper posterior part of the nasal cavity, the BBB does not exist. Therefore, substances introduced into the upper part of the nasal cavity can directly enter the brain. Access to the CNS is also possible through the optic nerve.

### The Embodiments

In one aspect of the invention, a system is provided for delivering a drug to the olfactory nerve, the system comprising:
(a) a source of the drug to be administered;
(b) a source of an excipient composition comprising eriodictyon fluid extract; and
(c) a pressurized delivery device, configured to deliver the drug and excipient to the olfactory nerve.

In an exemplary embodiment, the pressurized device is a bag-on-valve device. Such a device is generally considered a type of aerosol dispenser. A flexible bag within the can has its open end sealingly connected to a valve housing of the aerosol valve. The product to be dispensed from the aerosol container is filled into the flexible bag within the container and a liquified propellant or compressed gas is filled into the aerosol container outside of the bag, between the bag outer wall and the inner wall of the can. When the aerosol valve is actuated, the propellant acts against the outer wall of the bag to force the product being dispensed out the aerosol valve to the environment outside the can. When the valve actuation cases, of course, the product dispensing ceases. A device for use with a formulation of the invention may include an optionally detachable delivery nozzle or head, which is configured to deliver the drug and excipient to the olfactory nerve. In operation, the nozzle is inserted into the nasal cavity to the proper depth and a charge of the drug/excipient composition is administered within the nasal cavity, traveling to at least the upper third of the nasal cavity where an amount of the drug effective to achieve a desired result is absorbed through the olfactory nerve into the CNS.

An exemplary nozzle of use in the system of the invention, and in practicing the invention has a length insertable into the nasal cavity of from about 1 cm to about 3 cm.

The system in use delivers the agent to the nasal cavity of a mammal. It is preferred that the agent be delivered to the olfactory area in the upper third of the nasal cavity and particularly to the olfactory epithelium in order to promote transport of the agent into the peripheral olfactory neurons rather than the capillaries within the respiratory epithelium. The invention prefers the transport of agents to the brain by means of the nervous system instead of the circulatory system so that potentially therapeutic agents that are unable to cross the blood-brain barrier from the bloodstream into the brain may be delivered to the brain.

In a particularly preferred embodiment, the amount of drug absorbed through the olfactory nerve from a formulation of the instant invention is greater than the amount absorbed through this nerve from a formulation that does not include the eriodictyon extract. More preferably, the amount of drug absorbed by the olfactory nerve from a formulation of the instant invention per unit time is greater than that absorbed from a formulation that does not include the eriodictyon extract. Still other preferred embodiments result in a lower frequency or a lesser degree of irritation of the nasal cavity and/or olfactory nerve upon administration of a formulation of the invention when compared to an analogous formulation that does not include the eriodictyon extract.

It is preferred that the agent is capable of at least partially dissolving in the fluids that are secreted by the mucous membrane that surround the cilia of the olfactory receptor cells of the olfactory epithelium in order to be absorbed into the olfactory neurons. Alternatively, the invention may combine the agent with a carrier and/or other substances that foster dissolution of the agent within nasal secretions. Useful adjuvants include lipids, e.g., GM-1, phosphatidylserine (PS), and emulsifiers such as polysorbate 80.

To further facilitate the transport of the agent into the olfactory system, the the present invention combines the agent with a substance, e.g., eriodictyon extract, that enhances the residence time of the agent in contact with the olfactory epithelium, thereby enhancing absorption of the agent through the olfactory epithelium. It is generally preferred that the additives promote the retention of the agent in contact with the peripheral olfactory receptor cells. These peripheral neurons provide a direct connection between the brain and the outside environment due to their role in odor detection.

If the inherent permeability of mucosal tissue to some drugs (e.g., steroids) is too low to permit therapeutic levels of drugs to pass through a reasonably sized area of unbroken mucosa, a permeation enhancer can be administered with such drugs. Accordingly, in such cases, the enhancer will be present in the drug/excipient formulation.

Thus, in another exemplary embodiment, the agent includes a permeation-enhancing agent that increases permeation of the drug into the olfactory or optic nerve. Permeation-enhancing agents are selected from the groups consisting of bile acids, bile salts, ionic, nonionic zwitterionic, anionic, cationic, gemini pair surfactants, phospholipids, alcohols, glycyrrhetinic acid and its derivatives, enamines, salicylic acid and sodium salicylate, acetoacetate glycerol esters, dimethylsulfoxide, n-methylpyrrolinidinone, cyclodextrins, medium chain fatty acids, short chain fatty acids, short and medium chain diglycerides, short and medium chain monoglycerides, short chain triglycerides, calcium chelators, amino acids, cationic amino acids, homopolymeric peptides, cationic peptides, n-acetylamino acids and their salts, degradative enzymes, fatty acid synthesis inhibitors, cholesterol synthesis inhibitors.

Permeation enhancers are screened on a case-by-case basis to determine the most suitable candidate. Various models have been studied, for example those of LeCluyse and Sutton (1997. "In vitro models for selection of development candidates. Permeability studies to define mechanisms of permeation enhancement" Advanced Drug Delivery Reviews, 23:163-183). *In vitro* methods with the EpiAirway model have proven to be valuable.

It is preferred that the permeation-enhancing agent is lipophilic in order to promote absorption into the olfactory neurons and through the olfactory epithelium. Among those permeation-enhancing agents that are lipophilic are lipids, e.g., gangliosides and phosphatidylserine (PS). Uptake of non-lipophilic agents, e.g., nerve growth factor (NGF), may be enhanced by the combination with a lipophilic substance.

In one embodiment of the invention, the drug may be combined with micelles comprised of lipophilic substances. Such micelles may modify the permeability of the nasal membrane and enhance absorption of the agent. Among the lipophilic micelles that are preferred are gangliosides, particularly GM-1 ganglioside, and phosphatidylserine (PS). The neurologic agent may be combined with one or several types of micelle substances.

Once the drug has crossed the olfactory epithelium, the invention further provides for transport of the neurologic agent along the olfactory neural pathway and generally into the CNS. The agent itself may be capable of movement within the olfactory system. In particular, neurotrophic and neuritogenic substances have demonstrated ready incorporation into nerve cell membranes and an affinity for nerve cell receptor sites. Indications are that these substances are naturally synthesized in tissues in response to neural stimulation and that they subsequently bind to receptors on neurons where they act as nerve growth promoting factors.

An exemplary drug delivery system of the invention contain a source of drug, a source of a pharmaceutically acceptable excipient composition containing eriodictyon fluid extract. Generally, the system is configured to deliver both the selected drug and the eriodictyon fluid to the desired area. The system also provides a means for propelling the agent and extract through the nasal cavity a sufficient distance to bring the agent and extract into operative contact with the olfactory nerve.

The drug/excipient composition contained within the device and delivered therefrom to the olfactory nerve may take a number of forms. The drug and extract may be delivered "neat," i.e., in the absence of additional liquid. Alternatively, the drug and extract may be dissolved, dispersed or suspended in a suitable pharmaceutically acceptable solvent. The excipient composition contains eriodictyon fluid extract as its active ingredient, and may or may not contain any additional components. Typically, the excipient composition will be eriodictyon fluid extract itself. In some cases, however, other carriers or vehicles as described above may be incorporated into the excipient composition as well. Still other components can be incorporated into the excipient composition, including preservatives, stabilizers, surfactants, coloring agents, and the like. The drug/excipient composition in the reservoir will typically contain on the order of 0.1 to 20.0 wt. %, more preferably about 0.1 to 10.0 wt. %, most preferably about 0.5 to 5.0 wt. %, eriodictyon fluid extract (i.e., relative to the total drug/excipient composition), with the amount of drug dependent on a variety of factors, including the desired rate of delivery, the desired dosage, the disease to be treated, the nature and activity of the drug, the desired effect, possible adverse reactions, the ability and speed of the agent selected to reach its intended target, and other factors within the particular knowledge of the patient and the physician.

The eriodictyon fluid extract itself is an excellent source of antioxidants and can serve as a preservative for oxidation-sensitive drug substances (Liu, J Nat. Prod. 55: 357-363 (1992); Arriaga-Giner, Z. *Naturforsch.* **43**: 5-6 (1988)). Moreover, the role of reactive oxygen species (ROS) in various disease states is well-recognized. Thus, even in the absence of a drug, delivery of the anti-oxidants of the eriodictyon fluid extract to the CNS through the olfactory nerve provides a modality for treating and preventing a condition in which oxidation, e.g., by ROS, is implicated. Thus, the present invention also provides for the delivery of anti-oxidants to the CNS by delivery of a composition containing these anti-oxidants to the olfactory nerve. Exemplary anti-oxidants are those typically found in eriodictyon fluid extract, e.g., flavones.

In still another preferred embodiment, the composition has anti-cancer chemotherapeutic properties. The properties can be derived from species present in the eriodictyon fluid extract, or they can be added to the extract.

As noted above, a wide variety of drugs (active agents) can be used in conjunction with the compositions and devices of the present invention. Preferred drugs within the classes identified earlier herein include but are not limited to the following: analgesics such as aspirin, ibuprofen and acetaminophen; opiates such as morphine; antibiotics such as sulfacetamide, clindamycin and erythromycin; anticholinergic agents such as scopolamine; antidiabetic agents such as insulin; antifungal agents such as nystatin and amphotericin B; antiviral agents such as amantadine and acyclovir; antihistamines such as diphenhydramine; antinauseants such as meclozine; antineoplastic agents such as fluorouracil and methotrexate; antipsychotics such as prochlorperazine; steroids such as hydrocortisone, progesterone, and estrogens, therapeutic peptides such as growth factors, antibodies, insulin; and compositions for treating drug withdrawal containing, e.g., a CNS stimulant such as caffeine and a serotonin antagonist such as dihydroergotamine or a salt thereof (such compositions are described U.S. Pat. No. 5,051,426).

Suitable active agents include, but are not limited to, psychopharmacological agents, such as (1) central nervous system depressants, e.g., general anesthetics (barbiturates, benzodiazepines, steroids, cyclohexanone derivatives, and miscellaneous agents), sedative-hypnotics (benzodiazepines, barbiturates, piperidinediones and triones, quinazoline derivatives, carbamates, aldehydes and derivatives, amides, acyclic ureides, benzazepines and related drugs, phenothiazines, etc.), central voluntary muscle tone modifying drugs (anticonvulsants, such as hydantoins, barbiturates, oxazolidinediones, succinimides, acylureides, glutarimides, benzodiazepines, secondary and tertiary alcohols, dibenzazepine derivatives, valproic acid and derivatives, GABA analogs, etc.), analgesics (morphine and derivatives, oripavine derivatives, morphinan derivatives, phenylpiperidines, 2,6-methane-3-benzazocaine derivatives, diphenylpropylamines and isosteres, salicylates, p-aminophenol derivatives, 5-pyrazolone derivatives, arylacetic acid derivatives, fenamates and isosteres, etc.) and antiemetics (anticholinergics, antihistamines, antidopaminergics, etc.), (2) central nervous system stimulants, e.g., analeptics (respiratory stimulants, convulsant stimulants, psychomotor stimulants), narcotic antagonists (morphine derivatives, oripavine derivatives, 2,6-methane-3-benzoxacine derivatives, morphinan derivatives) nootropics, (3) psychopharmacologicals, e.g., anxiolytic sedatives (benzodiazepines, propanediol carbamates) antipsychotics (phenothiazine derivatives, thioxanthine derivatives, other tricyclic compounds, butyrophenone derivatives and isosteres, diphenylbutylamine derivatives, substituted benzamides, arylpiperazine derivatives, indole derivatives, etc.), antidepressants (tricyclic compounds, MAO inhibitors, etc.), pharmacodynamic agents, such as (1) peripheral nervous system drugs, e.g., local anesthetics (ester derivatives, amide derivatives), (2) drugs acting at synaptic or neuroeffector junctional sites, e.g., cholinergic agents, cholinergic blocking agents, neuromuscular blocking agents, adrenergic agents, antiadrenergic agents, (3) smooth muscle active drugs, e.g., spasmolytics (anticholinergics, musculotropic spasmolytics), vasodilators, smooth muscle stimulants, (4) histamines and antihistamines, e.g., histamine and derivative thereof (betazole), antihistamines (H₁-antagonists, H₂-antagonists), histamine metabolism drugs, chemotherapeutic agents, such as (1) anti-infective agents, e.g., ectoparasiticides (chlorinated hydrocarbons, pyrethins, sulfurated compounds), anthelmintics, antiprotozoal agents, antimalarial agents, antiamebic agents, antileiscmanial drugs, antitrichomonal agents, antitrypanosomal agents, sulfonamides, antimycobacterial drugs, antiviral chemotherapeutics, etc., and (2) cytostatics, i.e., antineoplastic agents or cytotoxic drugs, such as alkylating agents, e.g., Mechlorethamine hydrochloride (Nitrogen Mustard, Mustargen, HN2), Cyclophosphamide (Cytovan, Endoxana), Ifosfamide (IFEX), Chlorambucil (Leukeran), Melphalan (Phenylalanine Mustard, L-sarcolysin, Alkeran, L-PAM), Busulfan (Myleran), Thiotepa (Triethylenethiophosphoramide), Carmustine (BiCNU, BCNU), Lomustine (CeeNU, CCNU), Streptozocin (Zanosar) and the like; plant alkaloids, e.g., Vincristine (Oncovin), Vinblastine (Velban, Velbe), Paclitaxel (Taxol), and the like; antimetabolites, e.g., Methotrexate (MTX), Mercaptopurine (Purinethol, 6-MP), Thioguanine (6-TG), Fluorouracil (5-FU), Cytarabine (Cytosar-U, Ara-C), Azacitidine (Mylosar, 5-AZA) and the like; antibiotics, e.g., Dactinomycin (Actinomycin D, Cosmegen), Doxorubicin (Adriamycin), Daunorubicin (duanomycin, Cerubidine), Idarubicin (Idamycin), Bleomycin (Blenoxane), Picamycin (Mithramycin, Mithracin), Mitomycin (Mutamycin) and the like, and other anticellular proliferative agents, e.g., Hydroxyurea (Hydrea), Procarbazine (Mutalane), Dacarbazine (DTIC-Dome), Cisplatin (Platinol) Carboplatin (Paraplatin), Asparaginase (Elspar) Etoposide (VePesid, VP-16-213), Amsarcrine (AMSA, m-AMSA), Mitotane (Lysodren), Mitoxantrone (Novatrone), and the like. Chemotherapeutic agents may be those which are for treatment of a disease or disorder in the brain or central nervous system of the patient but demonstrate unacceptable systemic toxicity at desired doses. The general systemic toxicity associated with therapeutic levels of such agents may be reduced by their administration via the olfactory and/or optical nerves. Contemplated as examples are cardiotoxic compounds that are useful therapeutics but are dose limited by cardiotoxicity

Suitable active agents include, but are not limited to: Antibiotics, such as: aminoglycosides, e.g., amikacin, apramycin, arbekacin, bambermycins, butirosin, dibekacin, dihydrostreptomycin, fortimicin, gentamicin, isepamicin, kanamycin, micronomcin, neomycin, netilmicin, paromycin, ribostamycin, sisomicin, spectinomycin, streptomycin, tobramycin, trospectomycin; amphenicols, e.g., azidamfenicol, chloramphenicol, florfenicol, and theimaphenicol; ansamycins, e.g., rifamide, rifampin, rifamycin, rifapentine, rifaximin; beta.-lactams, e.g., carbacephems, carbapenems, cephalosporins, cehpamycins, monobactams, oxaphems, penicillins; lincosamides, e.g., clinamycin, lincomycin; macrolides, e.g., clarithromycin, dirthromycin, erythromycin, etc.; polypeptides, e.g., amphomycin, bacitracin, capreomycin, etc.; tetracyclines, e.g., apicycline, chlortetracycline, clomocycline, etc.; synthetic antibacterial agents, such as 2,4-diaminopyrimidines, nitrofurans, quinolones and analogs thereof, sulfonamides, sulfones;

Suitable active agents include, but are not limited to: Antimalarials, such as: acedapsone, amodiaquin, arteether, artemether, artemisinin, artesunate, atovaquone, bebeerine, berberine, chirata, chlorguanide, chloroquine, chlorprogaunil, cinchona, cinchonidine, cinchonine, cycloguanil, gentiopicrin, halofantrine, hydroxychloroquine, mefloquine hydrochloride, 3-methylarsacetin, pamaquine, plasmocid, primaquine, pyrimethamine, quinacrine, quinidine, quinine, quinocide, quinoline, dibasic sodium arsenate;

Suitable active agents include, but are not limited to: Antiprotozoan agents, such as: acranil, tinidazole, ipronidazole, ethylstibamine, pentamidine, acetarsone, aminitrozole, anisomycin, nifuratel, tinidazole, benzidazole, suramin, and the like.
Suitable drugs for use as active agents are also listed in: Goodman & Gilman's, The Pharmacological Basis of Therapeutics (9th Ed) (Goodman et al. eds) (McGraw-Hill) (1996); and 2006 Physician's Desk Reference.

Suitable active agents include, but are not limited to: antineoplastic agents, as disclosed in U.S. Pat. Nos. 5,880,161, 5,877,206, 5,786,344, 5,760,041, 5,753,668, 5,698,529, 5,684,004, 5,665,715, 5,654,484, 5,624,924, 5,618,813, 5,610,292, 5,597,831, 5,530,026; 5,525,633, 5,525,606, 5,512,678, 5,508,277, 5,463,181, 5,409,893, 5,358,952, 5,318,965, 5,223,503, 5,214,068, 5,196,424, 5,109,024, 5,106,996, 5,101,072, 5,077,404, 5,071,848, 5,066,493, 5,019,390, 4,996,229, 4,996,206, 4,970,318, 4,968,800, 4,962,114, 4,927,828, 4,892,887, 4,889,859, 4,886,790, 4,882,334, 4,882,333, 4,871,746, 4,863,955, 4,849,563, 4,845,216, 4,833,145, 4,824,955, 4,785,085, 4,684,747, 4,618,685, 4,611,066, 4,550,187, 4,550,186, 4,544,501, 4,541,956, 4,532,327, 4,490,540, 4,399,283, 4,391,982, 4,383,994, 4,294,763, 4,283,394, 4,246,411, 4,214,089, 4,150,231, 4,147,798, 4,056,673, 4,029,661, 4,012,448; psychopharmacological/psychot- ropic agents, as disclosed in U.S. Pat. Nos. 5,192,799, 5,036,070, 4,778,800, 4,753,951, 4,590,180, 4,690,930, 4,645,773, 4,427,694, 4,424,202, 4,440,781, 5,686,482, 5,478,828, 5,461,062, 5,387,593, 5,387,586, 5,256,664, 5,192,799, 5,120,733, 5,036,070, 4,977,167, 4,904,663, 4,788,188, 4,778,800, 4,753,951, 4,690,930, 4,645,773, 4,631,285, 4,617,314, 4,613,600, 4,590,180, 4,560,684, 4,548,938, 4,529,727, 4,459,306, 4,443,451, 4,440,781, 4,427,694, 4,424,202, 4,397,853, 4,358,451, 4,324,787, 4,314,081, 4,313,896, 4,294,828, 4,277,476, 4,267,328, 4,264,499, 4,231,930, 4,194,009, 4,188,388, 4,148,796, 4,128,717, 4,062,858, 4,031,226, 4,020,072, 4,018,895, 4,018,779, 4,013,672, 3,994,898, 3,968,125, 3,939,152, 3,928,356, 3,880,834, 3,668,210; analgesic agents, as disclosed in U.S. Pat. Nos. 5,292,736, 5,688,825, 5,554,789, 5,455,230, 5,292,736, 5,298,522, 5,216,165, 5,438,064, 5,204,365, 5,017,578, 4,906,655, 4,906,655, 4,994,450, 4,749,792, 4,980,365; 4,794,110, 4,670,541, 4,737,493, 4,622,326, 4,536,512, 4,719,231, 4,533,671, 4,552,866, 4,539,312, 4,569,942, 4,681,879, 4,511,724, 4,556,672, 4,721,712, 4,474,806, 4,595,686, 4,440,779, 4,434,175, 4,608,374, 4,395,402, 4,400,534, 4,374,139, 4,361,583, 4,252,816, 4,251,530, 5,874,459, 5,688,825, 5,554,789, 5,455,230, 5,438,064, 5,298,522, 5,216,165, 5,204,365, 5,030,639, 5,017,578, 5,008,264, 4,994,450, 4,980,365, 4,906,655, 4,847,290, 4,844,907, 4,794,110, 4,791,129, 4,774,256, 4,749,792, 4,737,493, 4,721,712, 4,719,231, 4,681,879, 4,670,541, 4,667,039, 4,658,037, 4,634,708, 4,623,648, 4,622,326, 4,608,374, 4,595,686, 4,594,188, 4,569,942, 4,556,672, 4,552,866, 4,539,312, 4,536,512, 4,533,671, 4,511,724, 4,440,779, 4,434,175, 4,400,534, 4,395,402, 4,391,827, 4,374,139, 4,361,583, 4,322,420, 4,306,097, 4,252,816, 4,251,530, 4,244,955, 4,232,018, 4,209,520, 4,164,514, 4,147,872, 4,133,819, 4,124,713, 4,117,012, 4,064,272, 4,022,836, 3,966,944; cholinergic agents, as disclosed in U.S. Pat. Nos. 5,219,872, 5,219,873, 5,073,560, 5,073,560, 5,346,911, 5,424,301, 5,073,560, 5,219,872, 4,900,748, 4,786,648; 4,798,841, 4,782,071, 4,710,508, 5,482,938, 5,464,842, 5,378,723, 5,346,911, 5,318,978, 5,219,873, 5,219,872, 5,084,281, 5,073,560, 5,002,955, 4,988,710, 4,900,748, 4,798,841, 4,786,648, 4,782,071, 4,745,123, 4,710,508; adrenergic agents, as disclosed in U.S. Pat. Nos. 5,091,528, 5,091,528, 4,835,157, 5,708,015, 5,594,027, 5,580,892, 5,576,332, 5,510,376, 5,482,961, 5,334,601, 5,202,347, 5,135,926, 5,116,867, 5,091,528, 5,017,618, 4,835,157, 4,829,086, 4,579,867, 4,568,679, 4,469,690, 4,395,559, 4,381,309, 4,363,808, 4,343,800, 4,329,289, 4,314,943, 4,311,708, 4,304,721, 4,296,117, 4,285,873, 4,281,189, 4,278,608, 4,247,710, 4,145,550, 4,145,425, 4,139,535, 4,082,843, 4,011,321, 4,001,421, 3,982,010, 3,940,407, 3,852,468, 3,832,470; antihistamine agents, as disclosed in U.S. Pat. Nos. 5,874,479, 5,863,938, 5,856,364, 5,770,612, 5,702,688, 5,674,912, 5,663,208, 5,658,957, 5,652,274, 5,648,380, 5,646,190, 5,641,814, 5,633,285, 5,614,561, 5,602,183, 4,923,892, 4,782,058, 4,393,210, 4,180,583, 3,965,257, 3,946,022, 3,931,197; steroidal agents, as disclosed in U.S. Pat. Nos. 5,863,538, 5,855,907, 5,855,866, 5,780,592, 5,776,427, 5,651,987, 5,346,887, 5,256,408, 5,252,319, 5,209,926, 4,996,335, 4,927,807, 4,910,192, 4,710,495, 4,049,805, 4,004,005, 3,670,079, 3,608,076, 5,892,028, 5,888,995, 5,883,087, 5,880,115, 5,869,475, 5,866,558, 5,861,390, 5,861,388, 5,854,235, 5,837,698, 5,834,452, 5,830,886, 5,792,758, 5,792,757, 5,763,361, 5,744,462, 5,741,787, 5,741,786, 5,733,899, 5,731,345, 5,723,638, 5,721,226, 5,712,264, 5,712,263, 5,710,144, 5,707,984, 5,705,494, 5,700,793, 5,698,720, 5,698,545, 5,696,106, 5,677,293, 5,674,861, 5,661,141, 5,656,621, 5,646,136, 5,637,691, 5,616,574, 5,614,514, 5,604,215, 5,604,213, 5,599,807, 5,585,482, 5,565,588, 5,563,259, 5,563,131, 5,561,124, 5,556,845, 5,547,949, 5,536,714, 5,527,806, 5,506,354, 5,506,221, 5,494,907, 5,491,136, 5,478,956, 5,426,179, 5,422,262, 5,391,776, 5,382,661, 5,380,841, 5,380,840, 5,380,839, 5,373,095, 5,371,078, 5,352,809, 5,344,827, 5,344,826, 5,338,837, 5,336,686, 5,292,906, 5,292,878, 5,281,587, 5,272,140, 5,244,886, 5,236,912, 5,232,915, 5,219,879, 5,218,109, 5,215,972, 5,212,166, 5,206,415, 5,194,602, 5,166,201, 5,166,055, 5,126,488, 5,116,829, 5,108,996, 5,099,037, 5,096,892, 5,093,502, 5,086,047, 5,084,450, 5,082,835, 5,081,114, 5,053,404, 5,041,433, 5,041,432, 5,034,548, 5,032,586, 5,026,882, 4,996,335, 4,975,537, 4,970,205, 4,954,446, 4,950,428, 4,946,834, 4,937,237, 4,921,846, 4,920,099, 4,910,226, 4,900,725, 4,892,867, 4,888,336, 4,885,280, 4,882,322, 4,882,319, 4,882,315, 4,874,855, 4,868,167, 4,865,767, 4,861,875, 4,861,765, 4,861,763, 4,847,014, 4,774,236, 4,753,932, 4,711,856, 4,710,495, 4,701,450, 4,701,449, 4,689,410, 4,680,290, 4,670,551, 4,664,850, 4,659,516, 4,647,410, 4,634,695, 4,634,693, 4,588,530, 4,567,000, 4,560,557, 4,558,041, 4,552,871, 4,552,868, 4,541,956, 4,519,946, 4,515,787,4,512,986, 4,502,989,4,495,102; the disclosures of all the above of which are herein incorporated by reference.

Cancer chemotherapeutic agents for use in the invention in principle include drugs which may be useful for treating brain tumors or other neoplasia in or around the brain. Such chemotherapeutic agents include adriamycin (also known as doxorubicin), cisplatin, paclitaxel, analogs thereof, and other chemotherapeutic agents demonstrate activity against tumours ex vivo and in vivo. Such chemotherapeutic agents also include alkylating agents, antimetabolites, natural products (such as vinca alkaloids, epidophyllotoxins, antibiotics, enzymes and biological response modifiers), topoisomerase inhibitors, microtubule inhibitors, spindle poisons, hormones and antagonists, and miscellaneous agents such as platinum coordination complexes, anthracendiones, substituted ureas, etc. Those of skill in the art will know of other chemotherapeutic agents.

The above disclosure provides basis for individual claims directed to any selected one of the drugs and drug classes mentioned.

In embodiments, the drug is not for use in treating a nasal condition, e.g. is not a decongestant such as , for example, oxymetazoline or is not an antiviral agent such as, for example, amantadine. In other embodiments the drug is not useful for treating a nasal or respiratory tract infection. Included also are embodiments in which the drug is not useful for treating a nasal or respiratory tract disease or disorder.

Included in the invention therefore are pharmaceutical formulations for delivery by nasal administration of a drug for the treatment of a disorder of the central nervous system to a mammalian nervous system, comprising:
the drug; and
an eriodictyon fluid extract.

The invention includes such formulations adapted for nasal administration. In embodiments, the drug is selected from the group consisting of analgesics; opiates; antibiotics; anticholinergic agents; antidiabetic agents; antinauseants; antineoplastic agents; antipsychotics; steroids, therapeutic peptides; compositions for treating drug withdrawal; psychopharmacological agents; peripheral nervous system drugs; drugs acting at synaptic or neuroeffector junctional sites; smooth muscle active drugs; histamines and antihistamines; anti-infective agents; cytostatics; antimalarials; antiprotozoan agents.

In more particular embodiments, the drug is selected from the group consisting of analgesics; opiates; antinauseants; antipsychotics; compositions for treating drug withdrawal; psychopharmacological agents; peripheral nervous system drugs; drugs acting at synaptic or neuroeffector junctional sites; smooth muscle active drugs.

In other particular embodiments, the drug is selected from the group consisting of antibiotics; antivirals; antimalarials; antiprotozoan agents.

It will be appreciated from the aforegoing that uses, compositions and systems are provided for the non-invasive transnasal and transocular drug delivery to the central nervous system using eriodictyon fluid extract technology. By administration through the olfactory nerve or the optical nerve, the delivery of a biologically active substance of interest into the CNS and CSF can be enhanced through bypassing the blood-brain barrier. The invention involves the use of eriodictyon fluid extract as an excipient in compositions and systems for administering drugs to the olfactory or optical nerve.

The pharmaceutical compositions and systems of the invention can be used in different ways to accomplish somewhat different purposes. The drug delivery device described above is preferred for administering drugs to the olfactory nerve via an intranasal administration route, and the eriodictyon fluid present in the excipient composition is useful in significantly reducing local irritation or sensitization that so frequently occurs with this type of drug delivery. Furthermore, the eriodictyon fluid facilitates adherence of the drug to the mucosal tissue.

In an exemplary embodiment, at least 60% of the uptake of an administered dose of the drug is taken-up by the nervous system, rather than the vasculature route (i.e., direct delivery to the nervous system, rather than systemic delivery through the vasculature, leading up to the nervous system uptake). In a preferred embodiment, at least 70% of the uptake of an administered dose of the drug is taken-up by the nervous system, rather than the vasculature route. In a more preferred embodiment, at least 80% of the uptake of an administered dose of the drug is taken-up by the nervous system, rather than the vasculature route. In an even more preferred embodiment, at least 90% of the uptake of an administered dose of the drug is taken-up by the nervous system, rather than the vasculature route. In the most preferred embodiment, at least 100% of the uptake of an administered dose of the drug is taken-up by the nervous system, rather than the vasculature route.

The materials, methods and devices of the present invention are further illustrated by the examples, which follow. These examples are offered to illustrate, but not to limit the claimed invention.

### EXAMPLES

### EXAMPLE 1

A solution of undisclosed active agent for treatment of a CNS disease for nasal administration is prepared as summarized in the following table:

| Components | Quantity |
|---|---|
| Water | 90.0 wt. % |
| Eriodictyon Fluid Extract | 2.5 wt. % |
| pH Buffer | 1.5 wt. % |
| Sodium Chloride | 5.0 wt. % |
| Active Agent | 1.0 wt. % |

The above composition is useful as a nasal spray. The composition may further include other active components as desired. The sodium chloride is added in an amount to adjust to the saline composition of normal nasal mucosal tissues, as is the pH of the composition.

### EXAMPLE 2

A formulation of eriodictyon fluid extract was prepared as summarized in the following table:

| Components | Quantity |
|---|---|
| Excipient (carrier) | 1.0-99 wt. % |
| Yerba Santa Fluid Extract* | 99-1.0 wt. % |
| Other Components | 0-9.0 wt. % |

| | |
|---|---|
| * Dried eriodictyon can be obtained from Meer Corporation, North Bergen, New Jersey. It can be used to prepare the fluid extract in a manner substantially as described in Remington's Pharmaceutical Sciences, 17th Ed., cited supra, on pages 1286 and 1516. After preparation of the extract, the above ingredients can be mixed to give a formulation of Yerba Santa extract. | |

It is understood that the examples and embodiments described herein are for illustrative purposes only and that various modifications or changes in light thereof will be suggested to persons skilled in the art and are to be included within the scope of the appended claims.

## Claims

1. A pharmaceutical formulation for delivery by nasal administration of a drug for the treatment of a disorder of the central nervous system comprising
an eriodictyon fluid extract and optionally a drug additional to the extract.

2. The formulation of claim 1 wherein the drug is selected from the group consisting of analgesics; opiates; antibiotics; anticholinergic agents; antidiabetic agents; antinauseants; antineoplastic agents; antipsychotics; steroids, therapeutic peptides; compositions for treating drug withdrawal; psychopharmacological agents; peripheral nervous system drugs; drugs acting at synaptic or neuroeffector junctional sites; smooth muscle active drugs; histamines and antihistamines; anti-infective agents; cytostatics; antimalarials; antiprotozoan agents.

3. The formulation of claim 1 or claim 2 wherein the drug is not useful for treating a nasal or respiratory tract infection, for example is not an antiviral agent useful for treating such infections or a decongestant.

4. The formulation of any preceding claim in combination with a pressurized delivery device, configured to deliver the drug and eriodictyon fluid extract to the olfactory nerve.

5. The use of an eriodictyon fluid extract for the manufacture of a medicament containing the eriodictyon fluid extract as an excipient or carrier and for the treatment of a disease of the central nervous system or brain.

6. The use of an eriodictyon fluid extract for the manufacture of a medicament containing a drug and the eriodictyon fluid extract as an excipient or carrier, for transnasal-mucosal delivery of the drug to a component of the nervous system, which component is a member selected from the peripheral nervous system, the central nervous system and combinations thereof.

7. The use of claim 6 wherein the drug delivery essentially bypasses the vasculature.

8. The use of an eriodictyon fluid extract for the manufacture of a medicament for administration by olfactory pathway(s) and containing a drug which is a component of the eriodictyon fluid extract or in addition to it as an excipient or carrier, the medicament being for delivery of the drug to a site in the brain or central nervous system.

9. The use of any of claims 5 to 8, wherein the medicament further comprises a solvent selected from the group consisting of deionized water, and an aqueous-based solution and a non-aqueous solvent.

10. The use of any of claims 5 to 8, wherein the medicament comprises an anti-oxidant or an anti-cancer chemotherapeutic.

11. The use of any of claims 5 to 8, wherein the medicament further comprises a permeation-enhancing agent to increase permeation of the drug into the olfactory nerve.

12. The use of any of claims 5 to 11, wherein the medicament further comprises a vasodilator, in addition to said drug.

13. The use of any of claims 5 to 11, wherein the medicament is for delivery of the drug to a basal forebrain, a prepyriform complex, a hippocampal formation, an amygdaloid nuclei, a nucleus basalis of Meynert, a locus ceruleus, a brainstem raphe nuclei, or a combination thereof.

14. An apparatus to enhance delivery of at least one delivery substance into the central nervous system of a mammal, utilizing the olfactory pathway(s) as a direct delivery route for the substance from an exogeneous source via the mammal's olfactory area to a target site of the mammal's central nervous system, bypassing the mammal's blood brain barrier comprising:
(a) a drug;
(b) an excipient composition comprising eriodictyon fluid extract; and
(c) a pressurized delivery device, configured to deliver the drug and excipient to the olfactory nerve.

15. The use of an eriodictyon fluid extract for the manufacture of a medicament containing the extract and the drug for providing enhanced delivery of a drug to a mammalian nervous system, optionally wherein the medicament is for administering said drug as a pharmaceutical composition with an eriodictyon fluid extract to a component of said nervous system, which is a member selected from the peripheral nervous system, the central nervous system and combinations thereof, transnasal-mucosally, essentially bypassing vasculature.

16. The use of claim 15, wherein: (i) the drug further comprises a solvent selected from the group consisting of deionized water, and an aqueous-based solution and a non-aqueous solvent; and/or
(ii) the drug comprises an anti-oxidant; and/or
(iii) the drug comprises an anti-cancer chemotherapeutic; and/or
(iv) the medicament further comprises a vasodilator; and/or
(v) the drug is absorbed into the olfactory system; and/or
((vi) a fibroblast growth factor included in the medicament, e.g. for transportion through a peripheral olfactory nerve into an olfactory bulb; and/or
(vii) the drug is for transporation to a basal forebrain, a prepyriform complex, a hippocampal formation, an amygdaloid nuclei, a nucleus basalis of Meynert, a locus ceruleus, a brainstem raphe nuclei, or a combination thereof.
